# EUROPEAN PATENT APPLICATION

(11) **EP 2 062 572 A1**
(43) Date of publication of application: **27.05.2009**
(21) Application number: 08253759.8
(22) Date of filing: 19.11.2008
(51) Int. Cl.: A61K 9/14, A61K 9/20, A61K 31/4196

(54) **Pharmaceutical compositions**

(30) Priority: 19.11.2007 US 3798 P; 22.01.2008 US 11949 P
(71) Applicant: Teva Pharmaceutical Industries Ltd., 49131 Petah Tiqva (IL)
(72) Inventor: Zadok, Uri, Herzliyya 46361 (IL); Gal, Yaron, Hadera (IL); Zalit, Ilan, Rosh Haain (IL)
(74) Representative: Wong, Kit Yee

(57) **Abstract**

The present invention provides a process of preparing a formulation with a sufficiently high dissolution rate and good bioavailability, which reduces the effect of the active material's physical characteristics on the chemical and physical properties of the final product.

## Description

### Cross Reference to Related Applications

The present invention claims the benefit of the following United States Provisional Patent Application Nos.: 61/003,798, filed November 19, 2007; and 61/011,949, filed January 22, 2008. The contents of these applications are incorporated herein by reference.

### Field of the Invention

The present invention relates to a pharmaceutical composition comprising a dry co-milled composition comprising a poorly water soluble active pharmaceutical ingredient (API) and at least two pharmaceutically acceptable excipients and a method of preparation thereof.

### Background of the Invention

Deferasirox has the chemical name 4-[3,5-bis(2-hydroxyphenyl)-[1,2,4]triazol-1-yl]benzoic acid, and is reported to have the following chemical structure:

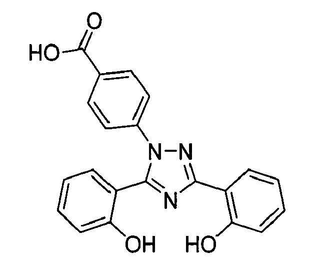

Deferasirox is an orally active iron chelator, currently marketed under the trade name EXJADE^{®} for the treatment of iron overload in transfusion dependent anemias (transfusion hemosiderosis), in particular thalassemia major, thalassemia intermediate and in sickle cell disease to reduce iron-related morbidity and mortality, in patients 2 years and older.

Deferasirox in the free acid form, salts thereof and its crystalline forms are described in the International Patent Publication WO 97/49395, which is hereby incorporated by reference.

Clinical thalassemias (major and intermediate) are hereditary disorders characterized by defective production of hemoglobin, which leads to decreased production and increased destruction of red blood cells.

Sickle cell disease is caused by a mutation in the hemoglobin-beta gene leading to the production of abnormal hemoglobin S, which causes rapid destruction of red blood cells (10-20 days instead of 120 days for normal red blood cells), thus causing anemia.

Hemochromatosis, the most common form of iron overload disease, is an inherited disorder that causes the body to absorb and store too much iron. The extra iron builds up in organs and damages them. Without treatment, the disease can cause these organs to fail.

Patients with sickle cell disease or thalassemia, who receive significant numbers of blood transfusions and patients with hematochromatosis require therapy to remove iron from the body, called chelation therapy.

Deferasirox is a once daily oral iron chelator, which is prescribed in the form of a dispersible tablet, i.e., a tablet which needs to be dispersed in an aqueous medium prior to administration. Dispersible tablets of deferasirox have been described in United States Application 2006/0110446 ("the '446 Application").

Deferasirox is typically administered at an initial dose of about 20 mg/kg body weight, and the dose is adjusted up to a maximum of 30 mg/kg body weight. Thus for an average adult patient weighing about 70 kg, the recommended daily dose is between 1.4 and 2.1 g of deferasirox. The total tablet weight for this dose is about 1.7 g.

International Patent Publication WO 2007/045445 describes dispersible tablets of deferasirox containing a maximum dose of 1000 mg of active material in the tablet.

The absorption and bioavailability of any particular therapeutic agent can be affected by numerous factors when dosed orally. One particular example is an oral dosage form where the active drug has low water solubility. In such cases, the particle size, the solubility (of the active pharmaceutical ingredient (API)), and the formulation used play an important role in the dissolution and consequently the bioavailability of the active drug substance. Usually, good bioavailability of such drugs requires a formulation which enables a good dispersion of the API in the gastrointestinal fluids. In a dosage form containing a high dose of API (above about 20% by weight of the formulation) the dependence of bioavailability on formulation increases, mainly due to the limited amount of inactive ingredients (which is limited by the maximum size of an oral dosage form). This leads to a stronger tendency of the API to agglomerate during the manufacturing process of the dosage form.

Several methods have been employed in an effort to alleviate the problems associated with the poor solubility of such active ingredients. For example, particle size reduction is often the method of choice. By reducing particle size, the effective surface area increases, thus allowing a greater dissolution rate. However, known shortcomings of particle size reduction are an increase in the tendency to agglomerate and an increase in static charge, reducing the tendency of the particles to flow and also leading to poor wetting properties. Another commonly used method is to dilute the active ingredient with large amounts of excipients. However, this approach is problematic when formulating dosage forms with high doses of active ingredients, due to the limit in size of the dosage forms.

All of these factors affect not only the production of the dosage form, but also the dissolution properties and bioavailability of the product.

Therefore, one of the main challenges in the development of formulations with a high load of low solubility drugs is to develop a formulation that eliminates the tendency of the API to agglomerate, thus increasing the bioavailability of the drug with reduced dependency on the API physical characteristics or source. Accordingly, there is a need for formulations and methods of their preparation that effectively increase the bioavailability following administration of such drugs.

### Summary of the Invention

In one embodiment, the present invention provides a method for producing a pharmaceutical composition comprising of a poorly water soluble active pharmaceutical ingredient (API), wherein said API is co-milled with at least two pharmaceutically acceptable excipients, to obtain a co-milled composition.

In another embodiment, the present invention provides a method for producing a pharmaceutical composition containing a poorly water soluble active pharmaceutical ingredient (API) or a pharmaceutically acceptable salt thereof, comprising dry co-milling said API with at least two pharmaceutically acceptable excipients to obtain a co-milled composition, wherein the pharmaceutically acceptable excipients are selected from the group comprising i) a surfactant and a sugar or sugar derivative, ii) a surfactant and a disintegrant, and iii) a combination thereof.

In another embodiment, the present invention provides a method for producing a pharmaceutical composition containing a poorly water soluble active pharmaceutical ingredient (API), comprising co-milling said API with at least two pharmaceutically acceptable excipients to obtain a co-milled composition; wherein the pharmaceutically acceptable excipients are selected from the group comprising i) a surfactant and a sugar or sugar derivative, ii) a surfactant and a disintegrant, and iii) a combination thereof.

Preferably, when the API is deferasirox, the pharmaceutical composition is in the form of a high-dose pharmaceutical composition and has a dissolution profile wherein at least about 60% by weight is dissolved in about 60 minutes.

In one embodiment the present invention provides a co-milled composition comprising a poorly water soluble active pharmaceutical ingredient (API) and at least two pharmaceutically acceptable excipients; wherein the API and the at least two pharmaceutically acceptable excipients are co-milled in a dry process and the pharmaceutically acceptable excipients are selected from the group comprising i) a surfactant and a sugar or sugar derivative, ii) a surfactant and a disintegrant, and iii) a combination thereof.

In another embodiment, the present invention provides a pharmaceutical composition comprising a dry co-milled composition comprising a poorly water soluble active ingredient (API) and at least two pharmaceutically acceptable excipient.

In a preferred embodiment, the above pharmaceutical composition is a high-dose pharmaceutical composition comprising about 20% to about 80% by weight of a poorly water soluble active pharmaceutical ingredient (API), preferably deferasirox or a pharmaceutically acceptable salt thereof, wherein said API is co-milled in a dry process with at least two pharmaceutically acceptable excipients, to obtain a co-milled composition. Preferably, the pharmaceutical composition is in a unit dosage form. The unit dosage form is preferably in the form of a tablet. The tablet may be in the form of an oral dosage form, or in the form of a dispersible tablet.

In a preferred embodiment the above pharmaceutical composition comprises the poorly soluble API deferasirox or a pharmaceutically acceptable salt thereof, a surfactant and a sugar or sugar derivative wherein: a.) the API is in an amount of about 20%-80% w/w; b.) the API is co-milled in a dry process with a surfactant, preferably sodium lauryl sulfate in an amount of less than about 1.75% by weight, and a sugar or sugar derivative; and c.) the formulation has a dissolution profile such that at least about 60% by weight is dissolved in 60 min.

### Brief Description of the Figures

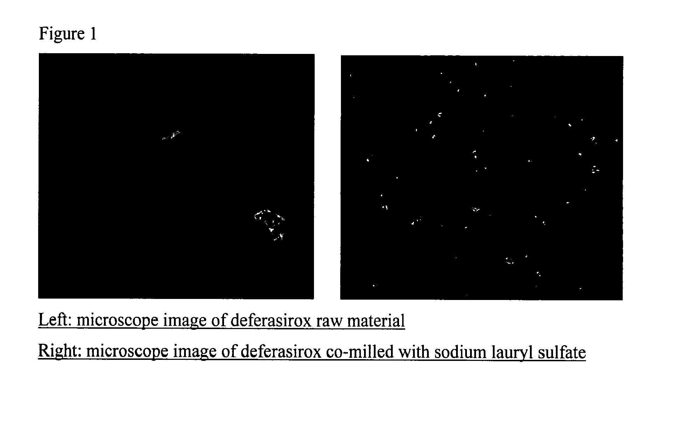

Figure 1: Microscope images of deferasirox raw material and deferasirox co-milled with sodium lauryl sulfate.

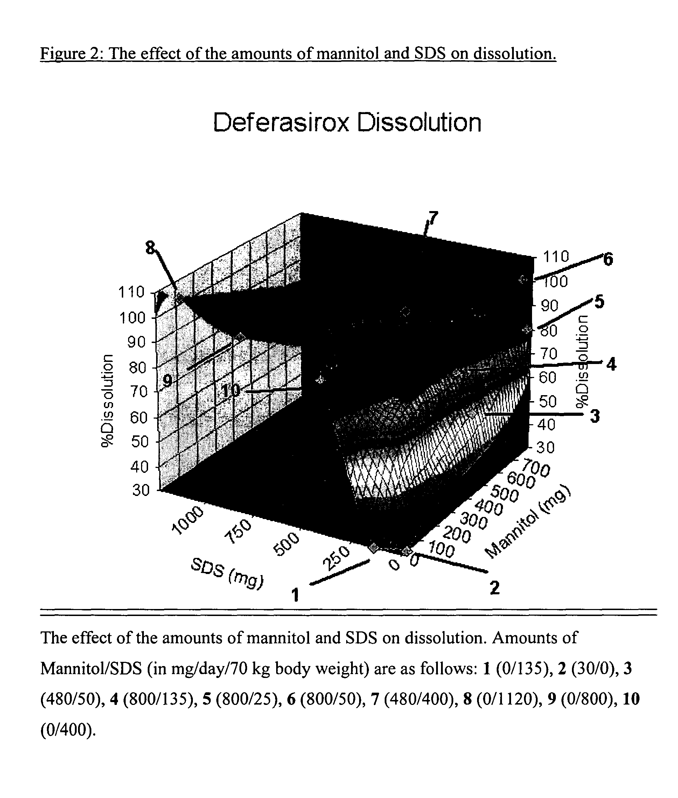

Figure 2: Illustration showing the effect of various amounts of mannitol and SDS on dissolution.

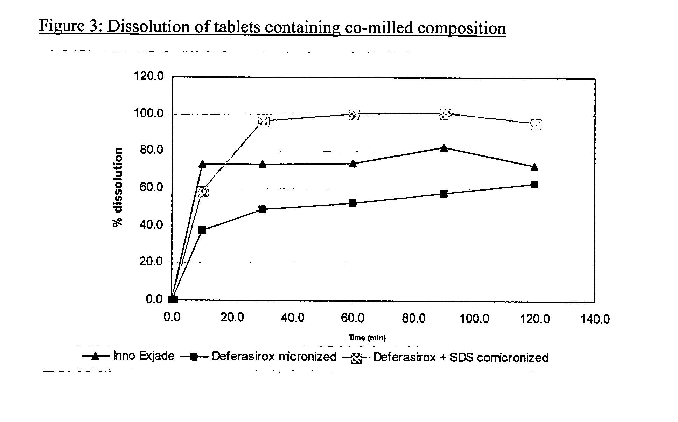

Figure 3: Dissolution of tablets containing co-milled composition.

### Detailed Description of the Invention

The present invention relates to pharmaceutical compositions and formulations of poorly water soluble drugs, with enhanced physicochemical characteristics, allowing for better processability, dissolution, and as a result, bioavailability for both high and low drug load composition.

As used herein the term "a dispersible tablet" refers to a tablet which has to be dispersed in an aqueous phase, e.g. water, prior to ingestion.

As used herein the terms "sodium lauryl sulfate" ("SLS") and "sodium dodecyl sulfate" ("SDS") are synonymous and are taken to be interchangeable.

As used herein the term "dissolution profile" refers to dissolution over time of the active ingredient from the pharmaceutical composition in simulated gastrointestinal fluid. Such dissolution profile can be determined by in an USP apparatus II (paddles), stirring at 50 r.p.m at 37°C, in an 0.05M phosphate buffer, pH 6, 0.15% sodium lauryl sulfate.

As used herein the term "% by weight" refers to a percentage by weight of the total pharmaceutical composition unless otherwise is indicated.

As used herein the term "poorly soluble" means having an aqueous solubility of preferably less than about 200 µg/mL at 37°C , more preferably less than about 100 µg/mL at 37°C, more preferably less than about 80 µg/mL at 37°C, more preferably less than about 50 µg/mL at 37°C.

As used herein the term "high-dose pharmaceutical composition" means a pharmaceutical composition or dosage form comprising more than about 20% by weight of active material. As used herein, "high-drug load" and "high-dose" when referring to a pharmaceutical composition are taken to be interchangeable.

As used herein the term "micronizing" includes reduction in size and deagglomeration. That term also includes treatment with a jet mill or treatment, while in a liquid dispersion, with a homogenizer, such as rotor-stator or high pressure homogenizer such as a Microfluidizer®. A jet mill uses compressed gas to produce comminuted particles from large dry particles. The mill is designed in such a way that the powdered particles exit the milling chamber and are collected in a collection vessel. According to a preferred embodiment, co-milling is carried out in an air jet mill for duration sufficient to reach a mean particle size of less than about 60µm, preferably less than 30µm, most preferably less than 20µm, for example between about 1µm and 40µm, between about 5µm and 15µm, between about 5µm and 10µm. Such co-milling may be carried out at a feed rate of about 400-500 mg/min, preferably about 450mg/min, a feed pressure of about 90-110 psi, preferably about 100 psi and a grinding pressure of about 70-90 psi, preferably about 80psi.

As used herein the term "co-micronizing" means milling a combination, e.g. of a mixture of more than one solid compound. As used herein, co-micronizing and co-milling are taken to be interchangeable.

Co-milling reduces the particulate size of the active ingredient and of the excipient, typically to a size smaller than 100µm, preferably less than 30µm, most preferably less than 20µm for example between about 1µm and 40µm, between about 5µm and 15µm, between about 5µm and 10µm, while creating particles which include an admixture of the active substance and the excipient, which are in intimate contact with each other.

As used herein the term "intimate contact" means an admixture of at least two components, in which there is no easy way to distinguish between the components, and there is no easy way to separate them. For the purposes of this invention the admixture of components which are in "intimate contact" behaves as a single component.

As used herein the term "dry" preferably means that no extraneous solvent is added.

In order to achieve the desired dissolution profile, co-milling with a pharmaceutically acceptable excipient may require such a high amount of said excipient such that the total daily intake would exceed the acceptable daily intake. Thus, although the dissolution profile of a particular pharmaceutical composition may be acceptable, the total daily intake of a surfactant (such as for example sodium lauryl sulfate), may exceed the acceptable daily intake. For example, with the formulations in Examples 17 and 18, where the dissolution after 60 minutes was 99% and 86%, respectively, the total daily intake of sodium lauryl sulfate with such formulations would be 416 mg and 320 mg (based on the assumption of an intake of 2000 mg of deferasirox), whereas the maximum (as defined by the FDA Inactive Ingredients Guide) is about 50 mg/day.

The present invention provides that, co-milling the active pharmaceutical ingredient in the preparation of a, preferably high-dose pharmaceutical composition, with 1) a surfactant, e.g. sodium lauryl sulfate, and a sugar or sugar derivative, e.g. a water soluble sugar or sugar derivative, e.g. lactose, or 2) a surfactant, e.g. sodium lauryl sulfate, and a disintegrant, e.g. crospovidone, is successful in providing an acceptable dissolution profile for the poorly water soluble API in a pharmaceutical composition, without exceeding the acceptable daily intake of the surfactant.

Additionally, co-milling as described above, greatly enhances the flow properties of the pharmaceutical composition, allowing the use of readily available pharmaceutical tableting machinery.

In a one embodiment, the present invention provides a method for producing a pharmaceutical composition comprising of a poorly water soluble active pharmaceutical ingredient (API) or a pharmaceutically acceptable salt thereof, wherein said API is co-milled with at least two pharmaceutically acceptable excipients, to obtain a co-milled composition.

In a preferred embodiment, the methods discussed herein is used for producing a high-dose pharmaceutical composition containing about 20% to about 80% by weight of a poorly water soluble active pharmaceutical ingredient (API), preferably deferasirox or a pharmaceutically acceptable salt thereof, and comprises co-milling said API in a dry process with at least two pharmaceutically acceptable excipients, to obtain a co-milled composition; and admixing the co-milled composition with at least one additional pharmaceutical excipient to obtain a high-dose pharmaceutical composition

The pharmaceutical excipients with which the API, preferably deferasirox or a pharmaceutically acceptable salt thereof is co-milled are preferably selected from the group comprising pharmaceutically acceptable surface active agents, disintegrants, and fillers or diluents, or their combinations. More preferably the API is co-milled with a pharmaceutically acceptable excipients or mixture of such excipients selected from the group consisting of i) a surfactant and a sugar or sugar derivative, ii) a surfactant and a disintegrant and iii) a combination thereof. Preferably, when the API is deferasirox, the pharmaceutical composition is in the form of a high-dose pharmaceutical composition and has a dissolution profile wherein at least about 60% by weight is dissolved in about 60 minutes.

Preferably the surface active agent (surfactant) is present in the co-milled composition (i.e. the co-milled composition of the API and the at least two pharmaceutically acceptable excipients) in an amount of about 0.25% to about 6%, more preferably about 0.25% to about 2.75% by weight, more preferably about 0.25% to about 2.5%, more preferably about 1.5-2.5% by weight of the total pharmaceutical composition.

In a preferred embodiment the surface active agent (surfactant) is present in the co-milled composition (i.e. the co-milled composition of the API and the at least two pharmaceutically acceptable excipients) in an amount of about 0.25% to about 5% by weight, more preferably about 0.5% to about 4.5%, more preferably about 0.8-4% by weight of the co-milled composition.

Preferably when the surface active agent (surfactant) is SDS it is present in the co-milled composition (i.e. the co-milled composition of the API and the at least two pharmaceutically acceptable excipients) in an amount of about less than 2%, about less than 1.75%, about 0.9% to about 1.75%, more preferably about 1.25% to about 1.75% by weight of the total pharmaceutical composition, more preferably about 1.5% to about 1.75%.

In a preferred embodiment the sugar or sugar derivative is selected from the group comprising mannitol, sorbitol, trehalose, lactose and sucrose or a combination thereof. Preferably the sugar is lactose (e.g. in the form of anhydrous lactose, or lactose monohydrate, and preferably spray dried lactose).

In another preferred embodiment the sugar or sugar derivative is present in the co-milled composition (i.e. the co-milled composition of the API and the at least two pharmaceutically acceptable excipients) in an amount of about 5% to about 75%, more preferably about 10% to about 60%, more preferably about 10% to about 45%, more preferably about 10% to about 40%, more preferably about 10% to about 35%, more preferably about 10% to about 25%, most preferably about 12% to about 20% by weight of the total pharmaceutical composition.

In a preferred embodiment the sugar or sugar derivative is present in the co-milled composition (i.e. the co-milled composition of the API and the at least two pharmaceutically acceptable excipients) in an amount of about 15% to about 60% by weight, more preferably about 15% to about 35%, more preferably about 17-30% by weight of the co-milled composition.

In a preferred embodiment the disintegrant is crospovidone.

In a preferred embodiment the disintegrant is present in an amount of about 2% to about 20%, more preferably about 4% to about 12% (for example, about 4% to about 9%), preferably about 5% to about 12%, more preferably about 7% to about 9%, most preferably about 8% to about 9% by weight of the total pharmaceutical composition.

In a preferred embodiment the disintegrant is present in the co-milled composition (i.e. the co-milled composition of the API and the at least two pharmaceutically acceptable excipients) in an amount of about 5% to about 25% by weight, more preferably about 5% to about 20%, more preferably about 7-16% by weight of the co-milled composition.

Preferably, the step of admixing the co-milled composition with at least one additional pharmaceutical excipient to obtain a pharmaceutical composition comprises blending the co-milled composition with at least one pharmaceutically acceptable excipient, optionally adding at least one pharmaceutically acceptable lubricant, and compressing the blend to a unit dosage form.

Preferably, the surface active agent is selected from the group comprising sodium lauryl sulfate and lauric acid. More preferably, the surface active agent is sodium lauryl sulfate. Preferably, the filler or diluent is selected from the group comprising water soluble sugars and sugar derivatives, e.g. sucrose, mannitol, lactose, water insoluble poly-sugars, such as cellulose derivatives, e.g. microcrystalline cellulose. More preferably, the filler or diluent is a water soluble sugar or sugar derivative. Even more preferably, the filler or diluent is mannitol or lactose. Most preferably, the filler or diluent is lactose. Preferably, the disintegrant is selected from the group comprising crospovidone, sodium starch glycolate, and croscarmellose sodium. More preferably, the disintegrant is crospovidone.

Preferably, the co-micronized composition comprises the API and a combination of an excipient selected from the group comprising disintegrants and fillers or diluents, and a surface active agent. More preferably, the co-micronized composition comprises the API and a combination of sodium lauryl sulfate, and a disintegrant or a filler or diluent. Most preferably, the co-micronized composition comprises the API and a combination of sodium lauryl sulfate and crospovidone or mannitol.

Co-milling may be performed using any of the methods known in the art. Preferably, the co-milling is effected using a jet-mill. Preferably, in the method of the present invention of preparing a pharmaceutical composition comprising a poorly water soluble active pharmaceutical ingredient (API) or a pharmaceutically acceptable salt thereof, the API is co-milled with at least one pharmaceutically acceptable excipient without the use of a solvent. In such preferred embodiments of the present invention co-milling is performed wherein each of the API and the at least one pharmaceutical acceptable excipient is in a solid form.

The present invention thus provides a method for producing a pharmaceutical composition of a poorly soluble active pharmaceutical ingredient (API), comprising the steps of (i) dry co-micronizing the API with at least two pharmaceutical excipient to form a co-micronized composition, and (ii) blending the co-micronized composition of part (i) with at least one pharmaceutically acceptable excipient, and incorporating said blend in a pharmaceutical dosage form.

In another embodiment, the present invention provides a pharmaceutical composition comprising a dry co-milled composition comprising a poorly water soluble active ingredient (API) and at least two pharmaceutically acceptable excipient and a method for producing the pharmaceutical composition.

In a preferred embodiment, the present invention provides a pharmaceutical composition of a poorly water soluble active pharmaceutical ingredient (API), preferably deferasirox or a pharmaceutically acceptable salt thereof, wherein the API is co-milled with at least two pharmaceutically acceptable excipients to obtain a co-milled composition; and admixing the co-milled composition with at least one additional pharmaceutical excipient to obtain a pharmaceutical composition. Preferably, when the API is deferasirox, the pharmaceutical composition is in the form of a high-dose pharmaceutical composition and has a dissolution profile wherein at least about 60% is dissolved in about 60 minutes. Preferably, the high-dose pharmaceutical composition is in a unit dosage form. The unit dosage form is preferably in the form of a tablet. The tablet may be in the form of an oral dosage form (e.g. for swallowing directly), or in the form of a dispersible tablet.

The high drug load pharmaceutical composition of the present invention comprises a poorly soluble API deferasirox and two pharmaceutically acceptable excipients wherein the pharmaceutically acceptable excipients are selected from the group consisting of: i) a surfactant and a sugar or sugar derivative, ii) surfactant and disintegrant, or iii) a combination thereof, wherein:
a) the API is present in an amount of about 20%-80% w/w;
b) the API is co-milled in a dry process with the excipients; and
c) the formulation has a dissolution profile such that at least about 60% is dissolved in 60 min.

In a preferred embodiment, the present invention provides a pharmaceutical composition comprising said co-milled composition, which is further blended with at least one pharmaceutically acceptable excipient, optionally adding at least one pharmaceutically acceptable lubricant, and, finally, compressing the blend to a unit dosage form. Preferably, the unit dosage form is in the form of a tablet. The tablet may be in the form of an oral dosage form (e.g. for swallowing directly), or in the form of a dispersible tablet.

Preferably, said co-milled pharmaceutical composition having a dissolution profile such that at least about 50% by weight of the API is dissolved within 30 minutes. Preferably, at least about 15% by weight is dissolved in 15 minutes and at least about 40% by weight within 30 minutes, preferably, at least 50% by weight is dissolved within 30 minutes. Dissolution profile is defined as above wherein the API is dissolved in a USP apparatus II (paddles), comprising 900 mL of liquid buffered to a pH of about 6, with about 0.15% SLS, at a temperature of about 37°C, and a rotation speed of about 50 rpm.

Preferably the poorly soluble API is deferasirox or pharmaceutically acceptable salts thereof. More preferably, deferasirox is in the form of its free acid.

In a preferred embodiment the API is present in the co-milled composition (i.e. the co-milled composition of the API and the at least two pharmaceutically acceptable excipients) in an amount of about 40%-70%, for example, 40%-65%, preferably about 45-55%, more preferably about 50% w/w of the total pharmaceutical composition.

In a preferred embodiment the API (e.g. desferasirox) is present in the co-milled composition (i.e. the co-milled composition of the API and the at least two pharmaceutically acceptable excipients) in an amount of about 25% to about 95% by weight, more preferably about 50%-90%, for example, 60%-80%, preferably about 65-75%, more preferably about 70% w/w of the co-milled composition.

In a preferred embodiment the surface active agent (surfactant) is selected from the group comprising sodium lauryl sulfate sodium (SLS), lauric acid, poloxamer or a combination thereof. Preferably, the surface active agent is selected from the group comprising sodium lauryl sulfate and lauric acid. More preferably, the surface active agent is sodium lauryl sulfate.

Preferably the surface active agent (surfactant( (e.g. sodium lauryl sulphate) is present in the co-milled composition (i.e. the co-milled composition of the API and the at least two pharmaceutically acceptable excipients) in an amount of about 0.25% to about 6%, more preferably about 0.25% to about 2.75% by weight, more preferably about 0.25% to about 2.5%, more preferably about 1.5-2.5% by weight of the total pharmaceutical composition.

Preferably when the surface active agent (surfactant) is SLS it is present in the co-milled composition (i.e. the co-milled composition of the API and the at least two pharmaceutically acceptable excipients) in an amount of about less than 2%, about less than 1.75%, about 0.9% to about 1.75%, more preferably about 1.25% to about 1.75% by weight, more preferably about 1.5% to about 1.75% of the total pharmaceutical composition.

In a preferred embodiment the sugar or sugar derivative is mannitol, sorbitol, trehalose, lactose or sucrose or a combination thereof. Preferably the sugar is lactose. In any embodiment of the process or compositions referred to herein, the sugar or sugar derivative preferably may be spray dried lactose.

In another preferred embodiment the sugar or sugar derivative (e.g. lactose) is present in the co-milled composition (i.e. the co-milled composition of the API and the at least two pharmaceutically acceptable excipients) in an amount of about 15% to about 60% by weight, more preferably about 15% to about 35%, more preferably about 17-30% by weight of the co-milled composition.

In another preferred embodiment the sugar or sugar derivative (e.g. lactose) is present in the co-milled composition (i.e. the co-milled composition of the API and the at least two pharmaceutically acceptable excipients) in an amount of about 5% to about 75%, more preferably about 10% to about 60%, more preferably about 10% to about 45%, more preferably about 10% to about 40%, more preferably about 10% to about 35%, more preferably about 10% to about 25%, most preferably about 12% to about 20% by weight of the total pharmaceutical composition.

In a preferred embodiment the disintegrant is selected from the group comprising alginic acid, microcrystalline cellulose, croscarmellose sodium, crospovidone, maltose, polacrilin potassium, sodium starch glycolate, or starch. Preferably, the disintegrant is crospovidone.

In a preferred embodiment the disintegrant (such as crospovidone) is present in an amount of about 2% to about 20%, more preferably about 4% to about 12% (for example, about 4% to about 9%), preferably about 5% to about 12%, more preferably about 7% to about 9%, most preferably about 8% to about 9% by weight of the total pharmaceutical composition.

In any of the embodiments of the present invention as described herein, the pharmaceutical composition further comprises at least one pharmaceutically acceptable excipient selected from the group comprising: glidant, lubricant, disintegrant, fillers and binders.

Preferably, when the pharmaceutical composition further comprises a disintegrant, the disintegrant, excluding the disintegrant included in the co-milled composition, is present in an amount of about 10%w/w or greater, more preferably about 15% to about 30%w/w, most preferably about 15% to about 26%w/w.

Preferably, when the pharmaceutical composition further comprises a binder, the binder is present in an amount greater than about 10%w/w, more preferably about 20% to about 30%w/w, most preferably about 20% to about 25%w/w.

Suitable glidants used in the formulation include, but are not limited to, colloidal silicon dioxide, talc, magnesium trisilicate, powdered cellulose, and calcium orthophosphate. Preferably, the pharmaceutically acceptable glidant is colloidal silicon dioxide. The glidant may be present in an amount from about 0.1 % to about 2% w/w, although these quantities may be adjusted to suit the specific formulation.

Suitable lubricants used in the formulation include, but are not limited to, stearic acid, magnesium stearate, zinc stearate, calcium stearate, magnesium trisilicate, and sodium stearyl fumarate. Preferably, the lubricant is selected from the group comprising sodium stearyl fumarate, stearic acid, magnesium stearate and zinc stearate. More preferably, the lubricant is magnesium stearate or sodium stearyl fumarate. Most preferably, the lubricant is magnesium stearate. The lubricant may be present in an amount from about 0.1 % to about 2%w/w, although these quantities may be adjusted to suit the specific formulation.

Suitable binders for the formulation include, but are not limited to, acacia, alginic acid, carbomer copolymer, carbomer interpolymer, copovidone, microcrystalline cellulose, dextrin, ethylcellulose, gelatin, glucose (liquid), guar gum, hydroxypropyl cellulose, maltose, methylcellulose, polyethylene oxide, polyvinylpyrrolidone, povidone, starch, or sodium carboxymethylcellulose. Preferred binders include polyvinylpyrrolidone or copovidone.

Suitable fillers for the formulation include, but are not limited to, microscrystalline cellulose, lactose, lactose monohydrate, spray dried lactose monohydrate, mannitol, starch, pregelatinized starch, dicalcium phosphate, modified celluloses. Preferred fillers are Microcrystalline cellulose, lactose, lactose monohydrate, and spray dried lactose monohydrate.

In any of the embodiments of the present invention as described herein, the surface of the API in the co-milled composition is in intimate contact with at least two excipients selected from the group consisting of surface-active agents and sugar or sugar derivative or disintegrant and/or a combination thereof.

In any of the embodiments of the present invention, the co-milled composition comprising particles of an API with low aqueous solubility, which is in intimate contact with a pharmaceutically acceptable excipients, selected from surface active agents, disintegrants, fillers or diluents, and their combinations.

The invention also provides a method for treating a medical condition by administering the high-dose composition of at least one of the above embodiments to a patient in need thereof. Examples of the medical conditions that may be treated include treatment of iron overload in transfusion dependent anemias (transfusion hemosiderosis), in particular thalassemia major, thalassemia intermediate and in sickle cell disease to reduce iron-related morbidity and mortality, in patients 2 years and older.

Having described the invention with reference to certain preferred embodiments, other embodiments will become apparent to one skilled in the art from consideration of the specification. The disclosures of the references referred to in this patent application are incorporated herein by reference. The invention is further defined by reference to the following examples describing in detail the process and compositions of the invention. It will be apparent to those skilled in the art that many modifications, both to materials and methods, may be practiced without departing from the scope of the invention.

### EXAMPLES

### EXAMPLE 1

2 g Deferasirox was mixed together with 0.1344 g sodium dodecyl sulfate for 5 minutes. The mixture was inserted into a Qualification Micronizer (Sturtevant, Hanover, Massachusetts) at a rate of 450 mg per minute, feed pressure 100 psi, grinding pressure 80 psi. The micronized material was collected into a filter bag. The yield was about 60 percent. The micronized composition was then used to prepare tablets as per Example 14.

### EXAMPLE 2

2 g Deferasirox was mixed together with 768 mg lactose spray dried as per Example 1. The micronized composition was then used to prepare tablets as per Example 15.

### EXAMPLE 3

2 g Deferasirox was mixed together with 480 mg mannitol as per Example
1. The micronized composition was then used to prepare tablets as per Example 20.

### EXAMPLE 4

7 g Deferasirox was mixed together with 1.68 g sodium starch glycolate as per Example 1. The micronized composition was then used to prepare tablets as per Example 22.

### EXAMPLE 5

7 g Deferasirox was mixed together with 0.896 mg sodium dodecyl sulfate and 2.8 g mannitol as per Example 1. The micronized composition was then used to prepare tablets as per Example 21.

### EXAMPLE 6

2 g Deferasirox was mixed together with 400 mg sodium dodecyl sulfate and 480 mg sodium starch glycolate as per Example 1. The micronized composition was then used to prepare tablets as per Example 23.

### EXAMPLE 7

2 g Deferasirox was inserted into a Qualification Micronizer (Sturtevant, Hanover, Massachusetts) at a rate of 450 mg per minute, feed pressure 100 psi, grinding pressure 80 psi. The micronized material was collected into a filter bag. The yield was about 60 percent. The micronized composition was then used to prepare tablets as per Example 16.

### EXAMPLE 8

2.5 g Deferasirox was inserted into a Qualification Micronizer (Sturtevant, Hanover, Massachusetts) together with 560 mg PEG 6000 at a rate of 450 mg per minute, feed pressure 100 psi, grinding pressure 80 psi. The micronized material was collected into a filter bag. The yield was about 60 percent. The micronized composition was then used to prepare tablets as per Example 31.

### EXAMPLE 9

2.5 g Deferasirox was inserted into a Qualification Micronizer (Sturtevant, Hanover, Massachusetts) together with 560 mg PEG 6000 and 62 mg of sodium dodecyl sulfate at a rate of 450 mg per minute, feed pressure 100 psi, grinding pressure 80 psi. The micronized material was collected into a filter bag. The yield was about 60 percent. The micronized composition was then used to prepare tablets as per Example 32.

### EXAMPLE 10

2.5 g Deferasirox was inserted into a Qualification Micronizer (Sturtevant, Hanover, Massachusetts) together with 420 mg crospovidone at a rate of 450mg per minute, feed pressure 100 psi, grinding pressure 80 psi. The micronized material was collected into a filter bag. The yield was about 60 percent. The micronized composition was then used to prepare tablets as per Example 33.

### EXAMPLE 11

2.5 g Deferasirox was inserted into a Qualification Micronizer (Sturtevant, Hanover, Massachusetts) together with 420 mg crospovidone and 62 mg of sodium dodecyl sulfate at a rate of 450 mg per minute, feed pressure 100 psi, grinding pressure 80 psi. The micronized material was collected into a filter bag. The yield was about 60 percent. The micronized composition was then used to prepare tablets as per Example 34.

### EXAMPLE 12

2.5 g Deferasirox was inserted into a Qualification Micronizer (Sturtevant, Hanover, Massachusetts) together with 200 mg croscarmellose sodium at a rate of 450mg per minute, feed pressure 100 psi, grinding pressure 80 psi. The micronized material was collected into a filter bag. The yield was about 60 percent. The micronized composition was then used to prepare tablets as per Example 35.

### EXAMPLE 13

2.5 g Deferasirox was inserted into a Qualification Micronizer (Sturtevant, Hanover, Massachusetts) together with 200 mg croscarmellose sodium and 62 mg of sodium dodecyl sulfate at a rate of 450 mg per minute, feed pressure 100 psi, grinding pressure 80 psi. The micronized material was collected into a filter bag. The yield was about 60 percent. The micronized composition was then used to prepare tablets as per Example 36.

### Examples 14-40 - Tablet Preparation

Dispersible tablets of deferasirox were prepared by preparing Phase Is as recited in Examples 1 to 13 and 48 to 49. The Phase Is was then inserted into a Qualification Micronizer (Sturtevant, Hanover, Massachusetts) at a rate of 450 mg per minute to give a co-milled composition. The co-milled composition was added to a blend formed from Phase II components. Phase III ingredients were then added to the above blend, and the final blend compacted to form a tablet with a diameter of about 10 mm.

| Dispersible Tablets Formulations (125mg, unless otherwise noted) | | | | | | | |
|---|---|---|---|---|---|---|---|
| | | Percent per dispersible tablet | | | | | |
| | | Ex. 14 | Ex. 15 | Ex.16 | Ex. 17 | Ex. 18 | |
| Components | | % | % | % | % | % | |
| Phase I | Deferasirox micronized | 50 | 50 | 50 | 46.3 | 50 | |
| | SDS | 3.36 | - | - | 25.93 | 20 | |
| | Lactose spray dried | - | 19.2 | - | - | - | |
| | Mannitol | - | - | - | - | - | |
| | Sodium starch glycolate | - | - | - | - | - | |
| Phase II | Crospovidone | 14 | 14 | 5 | 7.41 | 8 | |
| | Lactose spray dried | 19.2 | | 30.9 | 7.41 | 8 | |
| | Aerosil 200 | 6 | 6 | 0.5 | 0.46 | 0.5 | |
| | Povidone K-30 | 6 | 6 | 3 | 2.78 | 3 | |
| | SDS | - | 3.36 | 0.1 | - | - | |
| | Microcrystalline cellulose | - | | 10 | 9.26 | 10 | |
| Phase III | Mg stearate | 1.44 | 1.44 | 0.5 | 0.46 | 0.5 | |
| | | Ex. 19 | Ex. 20 | Ex. 21 | Ex. 22 | Ex. 23 | Ex. 24 |

| Components | | % | % | % | % | % | % |
|---|---|---|---|---|---|---|---|
| Phase I | Deferasirox micronized | 50 | 50 | 50 | 50 | 50 | 50 |
| | SDS | 10 | - | 10 | - | 10 | - |
| | Lactose spray dried | - | - | - | - | - | - |
| | Mannitol | - | 12 | 12 | - | - | - |
| | Sodium starch glycolate | - | - | - | 12 | 12 | - |
| Phase II | Crospovidone | 16 | 18 | 16 | 8 | 4 | 16 |
| | Lactose spray dried | 8 | 8 | 8 | 8 | 8 | 8 |
| | Aerosil 200 | 0.5 | 0.5 | 0.5 | 0.5 | 0.5 | 0.5 |
| | Povidone K-30 | 3 | 3 | 3 | 3 | 3 | 3 |
| | SDS | - | - | - | - | 10 | - |
| | Microcrystalline cellulose (Avicel PH-102) | 12 | 8 | - | 18 | 12 | 12 |
| Phase III | Mg stearate | 0.5 | 0.5 | 0.5 | 0.5 | 0.5 0.5 | 0.5 |
| | | Ex. 25 | Ex. 26 | Ex. 27 | Ex. 28 | Ex. 29 | Ex. 30 |

| Components | | % | % | % | % | % | % |
|---|---|---|---|---|---|---|---|
| Phase I | Deferasirox micronized | 50 | 50 | 50 | 50 | 50 | 50 |
| | SDS | 0.64 | 1.24 | 1.24 | 2.5 | - | - |
| | Lactose spray dried | | | | | | |
| | Mannitol | 20 | 20 | 12 | 12 | - | - |
| | Sodium starch glycolate | - | - | - | - | 8 | 20 |
| Phase II | crospovidone | 4.8 | 4.4 | 8 | 8 | 12 | 8 |
| | Lactose spray dried | 8 | 8 | 10 | 8.74 | 11.24 | 7.24 |
| | Aerosil 200 | 1.06 | 1.06 | 1.06 | 1.06 | 1.06 | 1.06 |
| | Povidone K-30 | 3 | 2.8 | 2.8 | 2.8 | 2.8 | 2.8 |
| | SDS | | | | | | |
| | Microcrystalline cellulose | 12 | 12 | 14.4 | 14.4 | 14.4 | 10.4 |
| Phase III | Mg stearate | 0.5 | 0.5 | 0.5 | 0.5 | 0.5 | 0.5 |
| | | Ex. 31 | Ex. 32 | Ex. 33 | Ex. 34 | Ex. 35 | Ex. 36 |

| Components | | % | % | % | % | % | % |
|---|---|---|---|---|---|---|---|
| Phase I | Deferasirox micronized | 50 | 50 | 50 | 50 | 50 | 50 |
| | SDS | - | 1.24 | - | 1.24 | - | 1.24 |
| | Polyethylene glycol 6000 | 11.2 | 11.2 | - | - | - | - |
| | Crospovidone | - | - | 8.4 | 8.4 | - | - |
| | Croscarmellose sodium | - | - | - | - | 4 | 4 |
| Phase II | Crospovidone | 14 | 12.8 | 16 | 16 | 18 | 17.2 |
| | Lactose spray dried | 7.24 | 7.24 | 7.24 | 7.2 | 7.24 | 7.2 |
| | Aerosil 200 | 1.06 | 1.02 | 1.06 | 1.06 | 1.06 | 1.06 |
| | Povidone k-30 | 2.8 | 2.8 | 2.8 | 2.8 | 2.8 | 2.8 |
| | Microcrystalline cellulose (Avicel PH-102) | 13.2 | 13.2 | 14 | 12.8 | 16.4 | 16 |
| Phase III | Mg stearate | 0.5 | 0.5 | 0.5 | 0.5 | 0.5 | 0.5 |
| | | Ex. 37 | Ex. 38 | Ex. 39 | Ex. 40* | | |

| Components | | % | % | % | % | | |
|---|---|---|---|---|---|---|---|
| Phase I | Deferasirox micronized | 50 | 50 | 50 | 50 | | |
| | SDS | 1.24 | 1.24 | 1.24 | 1.24 | | |
| | Lactose spray dried | 20 | | | 20 | | |
| | Sucrose | | 20 | | | | |
| | Sorbitol | | | 20 | | | |
| Phase II | crospovidone | 4.4 | 4.4 | 4.4 | 4.4 | | |
| | Lactose spray dried | 8 | 8 | 8 | 8 | | |
| | Aerosil 200 | 1.06 | 1.06 | 1.06 | 1.06 | | |
| | Povidone k-30 | 2.8 | 2.8 | 2.8 | 2.8 | | |
| | Microcrystalline cellulose (Avicel ph- | | | | | | |
| | 102) | 12 | 12 | 12 | 12 | | |
| Phase III | Mg stearate | 0.5 | 0.5 | 0.5 | 0.5 | | |

| | | | | | | | |
|---|---|---|---|---|---|---|---|
| * Tablets containing 500 mg deferasirox. | | | | | | | |

### EXAMPLE 41

3.75 g Deferasirox was inserted into a Qualification Micronizer (Sturtevant, Hanover, Massachusetts) together with 1.5 g lactose spray dried and 93 mg of sodium dodecyl sulfate at a rate of 450 mg per minute, feed pressure 100 psi, grinding pressure 80 psi. The micronized material was collected into a filter bag. The yield was about 60 percent. The micronized composition was then used to prepare tablets as per Example 37.

### EXAMPLE 42

3.75 g Deferasirox was inserted into a Qualification Micronizer (Sturtevant, Hanover, Massachusetts) together with 1.5 g Sucrose and 93 mg of sodium dodecyl sulfate at a rate of 450 mg per minute, feed pressure 100 psi, grinding pressure 80 psi. The micronized material was collected into a filter bag. The yield was about 60 percent. The micronized composition was then used to prepare tablets as per Example 38.

### EXAMPLE 43

3.75 g Deferasirox was inserted into a Qualification Micronizer (Sturtevant, Hanover, Massachusetts) together with 1.5 g sorbitol and 93 mg of sodium dodecyl sulfate at a rate of 450 mg per minute, feed pressure 100 psi, grinding pressure 80 psi. The micronized material was collected into a filter bag. The yield was about 60 percent. The micronized composition was then used to prepare tablets as per Example 39.

### EXAMPLE 44

10 g Deferasirox was inserted into a Qualification Micronizer (Sturtevant, Hanover, Massachusetts) together with 4 g lactose spray dried and 248 mg of sodium dodecyl sulfate at a rate of 450 mg per minute, feed pressure 100 psi, grinding pressure 80 psi. The micronized material was collected into a filter bag. The yield was about 60 percent. The micronized composition was then used to prepare tablets as per Example 40

### EXAMPLE 45

Deferasirox dissolution was carried out in a USP apparatus II (paddles). Test was conducted in 900 mL of 0.043M phosphate buffer, pH 6, 0.15% SDS at 37°C.

Paddle rotating speed 50 RPM. Samples were drawn at 10, 30, 60, 90, and 120 minutes.

The table below shows the percent of deferasirox dissolved after 60 minutes from the beginning of the experiment:

| Example # | % dissolved 30 min | % dissolved 60 min |
|---|---|---|
| Exjade® | 73 | |
| 14 | 31 | 35 |
| 15 | 23 | 25 |
| 16 | 21 | 25 |
| 17 | 112 | 106 |
| 18 | 91 | 99 |
| 19 | 79 | 86 |
| 20 | 24 | 28 |
| 21 | 94 | 97 |
| 22 | 52 | 66 |
| 23 | 41 | 44 |
| 24 | 40 | 42 |
| 25 | 81 | 79 |
| 26 | 81 | 82 |
| 27 | 56 | 59 |
| 28 | 70 | 73 |
| 29 | 33 | 36 |
| 30 | 34 | 39 |
| 31 | N/A | 33 |
| 32 | N/A | 35 |
| 33 | N/A | 74 |
| 34 | N/A | 84 |
| 35 | N/A | 51 |
| 36 | N/A | 61 |
| 37 | 96 | 97 |
| 38 | 63 | 74 |
| 39 | 86 | 91 |

| | | |
|---|---|---|
| N/A - not applicable, measurement was not performed. | | |

The percentage of deferasirox in tablets prepared according to Example 40 was 56% after 60 minutes employing the same conditions as above, since the dose was higher than in the previous examples (125mg vs. 500 mg). At these conditions, 44% of deferasirox from Exjade® 500mg tablets was dissolved after 60 minutes.

### Example 46 - Flow properties of co-milled blends

Flow properties were assessed visually, and given a score between 1 and 6, where 6 is a free-flowing powder. The results are summarized in the table below

| Co-milled composition | Flow score |
|---|---|
| Deferasirox raw material | 1 |
| Deferasirox, SDS | 2 |
| Deferasirox, lactose spray dried | 2 |
| Deferasirox, mannitol | 3 |
| Deferasirox, mannitol, SDS | 4 |
| Deferasirox, sodium starch glycolate | 3 |
| Deferasirox, sodium starch glycolate, SDS | 3 |
| Deferasirox, PEG 6000 | 2 |
| Deferasirox, PEG 6000, SDS | 2 |
| Deferasirox, crospovidone | 4 |
| Deferasirox, crospovidone, SDS | 5 |
| Deferasirox, croscarmellose sodium | 3 |
| Deferasirox, croscramellose sodium, SDS | 4 |

### Example 47 - Wet granulation

Blend 2500 mg of deferasirox, 420 mg crospovidone, and 62 mg sodium lauryl sulfate. Co-mill the blend using a Qualification Micronizer. Blend the co-milled composition with 140 mg polyvinyl pyrrolidone, and add water until a homogeneous granulate is obtained. Dry and mill the granulate, and add crospovidone, lactose, microcrystalline cellulose, colloidal silicon dioxide, and blend. Add magnesium stearate, and compact to form a tablet.

### Example 48 - Dry granulation

Blend 2500 mg of deferasirox, 420 mg crospovidone, and 62 mg sodium lauryl sulfate. Co-mill the blend using a Qualification Micronizer. Blend the co-milled composition with microcrystalline cellulose, and compact. Mill the compacted composition, add polyvinyl pyrrolidone, crospovidone, lactose, microcrystalline cellulose, colloidal silicon dioxide, and blend. Add magnesium stearate, and compact to form a tablet.

### EXAMPLE 49

2 g Deferasirox was mixed together with 1.037 g sodium dodecyl sulfate for 5 minutes. The mixture was inserted into a Qualification Micronizer (Sturtevant, Hanover, Massachusetts) at a rate of 450 mg per minute, feed pressure 100 psi, grinding pressure 80 psi. The micronized material was collected into a filter bag. The yield was about 60 percent. The micronized composition was then used to prepare tablets as per Example 17.

### EXAMPLE 50

2 g Deferasirox was mixed together with 0.8 g sodium dodecyl sulfate for 5 minutes. The mixture was inserted into a Qualification Micronizer (Sturtevant, Hanover, Massachusetts) at a rate of 450 mg per minute, feed pressure 100 psi, grinding pressure 80 psi. The micronized material was collected into a filter bag. The yield was about 60 percent. The micronized composition was then used to prepare tablets as per Example 18.

## Claims

1. A method for producing a pharmaceutical composition containing a poorly water soluble active pharmaceutical ingredient (API), comprising co-milling in a dry process said API with at least two pharmaceutically acceptable excipients to obtain a co-milled composition; wherein the pharmaceutically acceptable excipients are selected from the group comprising i) a surfactant and a sugar or sugar derivative, ii) a surfactant and a disintegrant, and iii) a combination thereof.

2. The method of claim 1, wherein the API is deferasirox or a pharmaceutically acceptable salt thereof.

3. The method of claim 2, wherein the API is deferasirox as a free acid.

4. The method of any of claims 1-3, wherein the API is present in an amount of about 20%-80% w/w.

5. The method of claim 4, wherein the API is present in an amount of about 50% w/w.

6. The method of any of claims 1 to 5, wherein the sugar or sugar derivative is mannitol, sorbitol, trehalose, lactose and sucrose or a combination thereof.

7. The method of claim 6, wherein the sugar or sugar derivative is mannitol or lactose.

8. The method of any of claims 1 to 7, wherein the sugar or sugar derivative is present in an amount of about 5% to about 75% by weight.

9. The method of claim 8, wherein the sugar or sugar derivative is present in an amount of about 12% to about 20% by weight

10. The method of any of claims 1 to 9, wherein the surface active agent (surfactant) is selected from the group comprising sodium lauryl sulfate (SLS), lauric acid, poloxamer or a combination thereof.

11. The method of claim 10, wherein the surface active agent is sodium lauryl sulfate.

12. The method of any of claims 1 to 11, wherein the surface active agent (surfactant) is present in an amount of about 0.25% to about 6% by weight.

13. The method of any of claims 1 to 12, wherein the surface active agent (surfactant) is SLS present in an amount of about less than 2% by weight

14. The method of claim 13, wherein SLS is present in an amount of about 1.25% to about 1.75% by weight.

15. The method of any of claims 1 to 14, wherein the disintegrant is crospovidone.

16. The method of any of claims 1 to 15, wherein the disintegrant is present in an amount of about 2% to about 20% by weight.

17. The method of claim 16, wherein the disintegrant is present in an amount of about 8% to about 9% by weight.

18. The method of any of claims 1 to 17, wherein co-milling is performed using a jet-mill.

19. The method of any of claims 1 to 18, further comprising dry admixing the co-milled composition and at least one additional pharmaceutical excipient to obtain a pharmaceutical composition; and compressing to produce a unit dosage form.

20. The method of claim 19, wherein the at least one additional pharmaceutical excipient is selected from the group comprising: a glidant, a lubricant, a disintegrant, and a filler or binder.

21. The method of claim 20, wherein the disintegrant, excluding any disintegrant included in the co-milled composition, is present in an amount greater than about 10% w/w.

22. The method of claim 21, wherein the disintegrant is present in an amount of about 15% to about 30% w/w.

23. A pharmaceutical composition comprising a poorly water soluble active pharmaceutical ingredient (API) prepared according to the method of any of claims 1 to 22.

24. The pharmaceutical composition of claim 23, wherein the API in the pharmaceutical composition has a dissolution profile such that at least about 60% by weight is dissolved in 60 min.

25. The pharmaceutical composition of claim 23 or claim 24 in the form of a tablet.

26. The pharmaceutical composition of claim 25, wherein the tablet is a dispersible tablet.

27. The pharmaceutical composition of any of claims 23 to 26 comprising the poorly soluble API deferasirox or a pharmaceutically acceptable salt thereof, a surfactant and a sugar derivative or a surfactant and a disintegrant wherein: a.) the API is in an amount of about 20%-80% w/w; b.) the API is co-milled in a dry process with a surfactant, preferably sodium lauryl sulfate in an amount of less than about 1.75% by weight, and a sugar or sugar derivative or a surfactant, preferably sodium lauryl sulfate in an amount of less than about 1.75% by weight, and a disintegrant; and c.) the composition has a dissolution profile such that at least about 60% by weight is dissolved in 60 min.

28. A pharmaceutical composition comprising a dry co-milled composition comprising a poorly water soluble active ingredient (API) and at least two pharmaceutically acceptable excipients.

29. The pharmaceutical composition of claim 28, wherein the API is deferasirox or a pharmaceutically acceptable salt thereof.

30. The pharmaceutical composition of claim 29, wherein deferasirox is in amount of about 20%-80% by weight.

31. The pharmaceutical composition of any of claims 28 to 30, wherein the pharmaceutical composition has a dissolution profile such that at least about 60% of the API is dissolved in 60 minutes.

32. The pharmaceutical composition of any of claims 28 to 31 comprising the poorly soluble API deferasirox or a pharmaceutically acceptable salt thereof, a surfactant and a sugar derivative or a surfactant and a disintegrant wherein: a.) the API is in an amount of about 20%-80% w/w; b.) the API is co-milled in a dry process with a surfactant, preferably sodium lauryl sulfate in an amount of less than about 1.75% by weight, and a sugar or sugar derivative or a surfactant, preferably sodium lauryl sulfate in an amount of less than about 1.75% by weight, and a disintegrant; and c.) the composition has a dissolution profile such that at least about 60% by weight is dissolved in 60 min.

33. A co-milled composition comprising a poorly water soluble active pharmaceutical ingredient (API) and at least two pharmaceutically acceptable excipients; wherein the API and the at least two pharmaceutically acceptable excipients are co-milled in a dry process and the pharmaceutically acceptable excipients are selected from the group comprising i) a surfactant and a sugar or sugar derivative, ii) a surfactant and a disintegrant, and iii) a combination thereof.

34. The composition of claim 33, wherein the API is deferasirox or a pharmaceutically acceptable salt thereof.

35. The composition of claim 34, wherein the API is deferasirox as a free acid.

36. The pharmaceutical composition of any of claims 23 to 32 for use in treating iron overload in transfusion dependent anemias (transfusion hemosiderosis), thalassemia major, and thalassemia intermediate or in reducing iron-related morbidity and mortality in sickle cell disease in patients 2 years and older, wherein the API is deferasirox.

37. The pharmaceutical composition of any of claims 23 to 32 in the manufacture of a medicament for treating iron overload in transfusion dependent anemias (transfusion hemosiderosis), thalassemia major, and thalassemia intermediate or for reducing iron-related morbidity and mortality in sickle cell disease in patients 2 years and older, wherein the API is deferasirox.
